(19) Europäisches Patentamt

European Patent Office

Office européen des brevets

(11) **EP 0 894 117 B1**

(12) **EUROPÄISCHE PATENTSCHRIFT**

(45) Veröffentlichungstag und Bekanntmachung des Hinweises auf die Patenterteilung:
**08.08.2001   Patentblatt 2001/32**

(51) Int Cl.[7]: **C08L 83/04**, A61K 6/10

(21) Anmeldenummer: **96914943.4**

(86) Internationale Anmeldenummer:
**PCT/EP96/01623**

(22) Anmeldetag: **18.04.1996**

(87) Internationale Veröffentlichungsnummer:
**WO 97/40102 (30.10.1997 Gazette 1997/46)**

(54) **ADDITIONSVERNETZENDE 2-KOMPONENTEN-SILICONMATERIALIEN**

ADDITION CROSS-LINKING, TWO-COMPONENT SILICONE MATERIALS

MATERIAUX SILICONES A DEUX CONSTITUANTS RETICULANTS PAR ADDITION

(84) Benannte Vertragsstaaten:
**CH DE ES FR GB IT LI**

(43) Veröffentlichungstag der Anmeldung:
**03.02.1999   Patentblatt 1999/05**

(73) Patentinhaber: **Kettenbach GmbH & Co KG**
**35713 Eschenburg (DE)**

(72) Erfinder:
 • **BUBLEWITZ, Alexander**
  **D-35745 Herborn (DE)**
 • **REBER, Jens-Peter**
  **D-35745 Herborn (DE)**

(74) Vertreter:
 **Meyers, Hans-Wilhelm, Dr.rer.nat.Dipl.-Chem.**
 **Patentanwälte**
 **von Kreisler-Selting-Werner,**
 **Bahnhofsvorplatz 1**
 **50667 Köln (DE)**

(56) Entgegenhaltungen:
 **EP-A- 0 305 073**          **EP-A- 0 522 341**
 **EP-A- 0 579 132**          **WO-A-93/17654**

Bemerkungen:
 Die Akte enthält technische Angaben, die nach dem Eingang der Anmeldung eingereicht wurden und die nicht in dieser Patentschrift enthalten sind.

## Beschreibung

In der Zahnmedizin und Zahntechnik werden bei vielen Maßnahmen Materialien benötigt, die aus einem viskosen Zustand in einen besonders harten Endzustand übergehen. Vor allem bei der Verschlüsselung, Fixierung, Positionierung, Wiederherstellung, Übertragung und Remontage besteht die Forderung an eine hohe Endhärte an das hierfür verwendete Material. Eines der wichtigen Anwendungsgebiete hierbei ist die Bißregistrierung.

Bei der Bißnahme soll die Lagebeziehung der beiden Kiefer zueinander genau fixiert werden. Das daraus hervorgehende Bißregistrat dient als Arbeitsgrundlage beim Zahntechniker, um die patientengerechte Bißsituation auf die Modellsituation zu übertragen. Bißregistrate werden vom Zahnarzt in fast allen Fachgebieten angefertigt. Folgende Anforderungen werden an ein geeignetes Bißregistriermaterial gestellt:

Für eine präzise Registrierung ist es wichtig, daß das Material keine Verschiebung in vertikaler und horizontaler Richtung zuläßt. Damit ist eine besonders hohe Endhärte und eine niedrige Elastizität des Materials eine zentrale Forderung. Außerdem ist die hohe Härte für eine gute Bearbeitung des Registrats mit einer Fräse oder einem Skalpell eine Voraussetzung. Für eine Kontrolle der richtigen Registrierung schon im Patientenmund ist es wünschenswert, überschüssiges Material gezielt abbrechen zu können. Bei der Registrierung wird eine besonders kurze Mundverweildauer bei einer ausreichenden Gesamtverarbeitungszeit gefordert, um ein Verschieben der Kiefer während der Abbindephase zu vermeiden. Das Material darf sich während des Transports von der Zahnarztpraxis ins Zahntechnikerlabor und darüber hinaus nicht in seiner Dimension verändern. Der Anwender wünscht ein einfaches Handling beim Dosieren, Mischen und Applizieren und eine möglichst lange Lagerstabilität des Materials.

Es ist bekannt additionsvernetzende Silicone für die Bißregistrierung einzusetzen; sie weisen eine höhere Endhärte und geringere elastische Verformung gegenüber üblichen Siliconen in der Zahnmedizin auf.

Von den auf dem Markt befindlichen Produkten werden Shore A-Endhärten von 70 - 90 erreicht.

Für die Indikation Bißregistrierung ist demnach eine wesentliche Steigerung der Endhärte bzw. des Elastizitätsmoduls für die Erhöhung der Präzision wünschenswert.

Das im Anschluß beschriebene A-Silicon mit besonders hoher Endhärte bringt z. B. für folgende Indikationen wesentliche Vorteile:

- spritzbares Material für die Bißregistrierung
- Material zur Beschichtung der Bißgabel oder Registrierplatten bei der Registrierung
- Schlüsselmaterial, z. B. für intraorale Stützstift-Registrate
- Übertragung von Brackets in der Kieferorthopädie
- Remontage von Keramik-, Guß- oder Kunststoffüllungen zum Einschleifen der Occlusion auf dem Modell
- hartwerdendes Unterfütterungssilicon
- Modellmaterial zur Herstellung von Inlays/Onlays.

Die oben angesprochenen bekannten additionsvernetzenden Silicone bestehen aus 2 Komponenten, die über eine edelmetallkatalysierte Hydrosilylierungs-Reaktion chemisch vernetzen.

Die Komponenten A und B sind in verschiedenen Viskositäten (dünn-, mittel-, zähfließend und knetbar) und Mischungsverhältnissen (in der Regel 1:1) einstellbar. Die Mischung der Komponenten A und B ist gewichts- und volumenmäßig durchführbar und erfolgt über geeignete Dosier- und Mischvorrichtungen, wie z. B. Stranglängendosierung und manuelles Mischen, Kartuschensystem mit statischem Mischer und elektrisch/mechanisch betriebene Mischgeräte. Nach dem Dosieren und Mischen der Komponenten A und B wird die resultierende, homogen gemischte Paste innerhalb der Gesamtverarbeitungszeit mit geeigneten Hilfsmitteln in den Patientenmund appliziert. Nach dem Aushärten des Materials (Mundverweildauer) wird dieses aus dem Patientenmund entnommen und nach Bedarf weiterverarbeitet.

Nach dem Stand der Technik weisen Bißregistriermaterialien auf Basis additionsvernetzender Silicone folgende Rohstoffzusammensetzung auf:

a) Organopolysiloxane mit zwei oder mehr Vinylgruppen im Molekül und einer Viskosität zwischen 100 - 350.000 MPa·s,

b) Organopolysiloxane der allgemeinen Formel: $CH_2=CH-SiR_2O-(SiR_2O)_n-SiR_2-CH=CH_2$, worin "R" gleiche oder verschiedene von aliphatischen Mehrfachverbindungen freie, einwertige, gegebenenfalls substituierte Kohlenwasserstoffreste und "n" eine ganze Zahl im Wert von 10 - 20 bedeutet,

c) niedermolekulare vinyl- und ethoxygruppenhaltige MQ-Harze,

d) Organohydrogenpolysiloxane (SiH-Vernetzer) mit zwei oder mehr SiH-Gruppen im Molekül,

e) Katalysatoren zur Beschleunigung der Hydrosilylierungs-reaktion,

f) Farbstoffe,

g) verstärkende Füllstoffe (ggf. auch oberflächenbehandelt, gefällt und pyrogen hergestellt),

h) Compounds aus Polydimethylsiloxanen und o. g. verstärkenden Füllstoffen,
i) nichtverstärkende Füllstoffe (ggf. auch oberflächenbehandelt).

Durch die EP-A-0 522 341 ist ein transparentes Material auf der Basis additionsvernetzender Polysiloxane bekannt, welches bevorzugt zur Bißregistrierung im Dentalbereich Anwendung findet, um eine Kontrolle von Einstellungen oder Positionierungen aufgrund ihrer Durchsichtigkeit zu erlauben und um eine Fixation durch dieses Material, z. B. durch Lichtpolymerisation, zu ermöglichen. Gegenstand sind hiernach bei Umgebungstemperatur härtbare Massen auf Polysiloxanbasis, die nach dem Additionsverfahren vernetzen und die neben Organopolysiloxane mit zwei oder mehr Vinylgruppen im Molekül, Organohydrogenpolysiloxane mit zwei oder mehreren Si-H-Gruppen im Molekül, einem Katalysator zur Beschleunigung der Additionsreaktion und ggf. Farbstoffe, wobei letztere die gewünschte Transparenz beeinträchtigen, noch Compounds von hochdispersen, aktiven Füllstoffen in Siliconöl, kurzkettige Organopolysiloxane mit zwei oder mehreren Vinylgruppen im Molekül und ggf. ein niedermolekulares, Vinyl- und Ethoxygruppenhaltiges, in dem Organopolysiloxane mit zwei oder mehr Vinylgruppen im Molekül homogen lösliches QM-Harz enthalten, welches erstens einen Vinylgruppengehalt von 0,5 - 8 mMol/g besitzt und zweitens aus $SiO_{4/2}$-, $RO_{1/2}$- und $R_3SiO_{1/2}$-Einheiten besteht und R für eine Methyl-, Vinyl-, Phenyl- und Ethylgruppe steht und einen Ethoxygruppengehalt von weniger als 4mMol/g aufweist, enthalten, wobei die Organohydrogenpolysiloxane mit zwei oder mehreren Si-H-Gruppen im Molekül in die Basispaste und der Katalysator zur Beschleunigung der Additionsreaktion in der Katalysatorpaste enthalten sind. Basis- und Katalysatorpaste sollen sich durch eine hohe Temperatur- und gute Lagerstabilität hinsichtlich Viskosität auszeichnen. Die aus dieser Masse hergestellten Bißregistrate sollen hohe Härte, hohe Transparenz und Reißfestigkeit bei geringer elastischer Verformung aufweisen. Die hier eingesetzten QM-Harze müssen dabei so niedermolekular sein, daß sie in den vinylendgestoppten Polydimethylsiloxanen optisch klar lösbar sind, um die gewünschte Transparenz der Bißregistrate zu erhalten. Die somit erhaltenen Produkte weisen keine-hohe Shore D-Härte (10-19) und E-Modul (~5-9 MPa) auf.

EP-A-0579132 und WO-A-93/17654 beschreiben dentale Abformmaterialen auf additionsvernetzender Siliconbasis.

Aufgabe der vorliegenden Erfindung ist es, additionsvernetzende 2-Komponenten-Siliconmaterialien mit hoher Shore D-Härte und hohem E-Modul zu schaffen und somit Zusammensetzungen von additionsvernetzenden Zweikomponenten-Siliconmaterialien zu erhalten, die in vulkanisiertem Zustand aufgrund ihrer hohen Shore D-Härte und ihres hohen Elastizitätsmoduls im Vergleich zu bekannten A-Siliconen besonders vorteilhaft für unterschiedliche Aufgabenstellungen in der Zahnheilkunde und in der Zahntechnik eingesetzt werden können, die bisher noch nicht zufriedenstellend gelöst sind.

Die hierfür geforderten Shore D-Härten von größer 35 und Elastizitätsmodule größer 20 MPa sollen unter Beibehaltung der sonstigen mechanischen und verarbeitungsfreundlichen Eigenschaften hinsichtlich sinnvoll verarbeitbarer Viskositäten/Konsistenzen mit den oben aufgeführten Dosier- und Mischsystemen, schnellem Abbindeverhalten, Flexibilität, Bearbeitbarkeit, Entformbarkeit, Fließverhalten während der Verarbeitungszeit und ggf. glatter Oberflächenbeschaffenheit, erzielt werden. Dabei soll die Formulierung der Inhaltsstoffe über eine ausreichende Lagerstabilität in der entsprechenden Primärverpackung, z. B. Kartusche oder Tube verfügen, wobei unter Lagerstabilität in diesem Zusammenhang beispielsweise eine Aufrechterhaltung der Leistungsmerkmale vor und während der Vulkanisation hinsichtlich der Viskosität/Konsistenz der Einzelkomponenten und der Mischung sowie der Abbindecharakteristik über mindestens 12 Monate und nach der Vulkanisation hinsichtlich der Shore Härte, Elastizität und des Elastizitätsmoduls über mindestens 1 Monat zu verstehen ist.

Die vorliegende Erfindung, wie in den Anprüchen definiert, löst diese Aufgabe und erfüllt die oben genannten Forderungen durch folgende Zusammensetzung von Inhaltsstoffen:

a) Organopolysiloxane mit zwei Vinylgruppen im Molekül
b) Organohydropolysiloxane mit zwei oder mehr SiH-Gruppen im Molekül
c) Katalysatoren zur Beschleunigung der Hydrosilylierungsreaktion
d) verstärkende Füllstoffe
e) nicht verstärkende Füllstoffe
f) gegebenenfalls Farbstoffe
g) gegebenenfalls Feuchtigkeitsbinder
h) gegebenenfalls Organopolysiloxane mit mehr als zwei Vinylgruppen im Molekül
i) gegebenenfalls Inhibitoren zur Reaktivitätseinstellung
j) gegebenenfalls vinylgruppenhaltige, feste oder flüssige MQ-Harze
k) gegebenenfalls Compounds aus Organopolysiloxanen und verstärkenden Füllstoffen
l) gegebenenfalls Tenside, Emulgatoren und Stabilisatoren
m) gegebenenfalls radioopake Substanzen
n) gegebenenfalls $H_2$-absorbierende bzw. adsorbierende Substanzen und Substanzen, die die $H_2$-Entwicklung

eliminieren bzw. reduzieren.

Die im nachfolgenden genannten Vorteile des erfindungsgemäßen Siliconmaterials werden vergleichend in Tabelle 1 und 2 dargestellt.

Das erfindungsgemäße Siliconmaterial zeichnet sich insbesondere durch eine hohe Shore D-Härte und durch ein hohes Elastizitätsmodul im vulkanisierten Zustand aus.

Aufgrund der hohen Shore D-Härte ist eine gezielte Brechbarkeit des im Mund ausgehärteten überschüssigen Siliconmaterials durchführbar, so daß eine visuelle Kontrolle der Bißnahme im Gegensatz zu EP-A-0 522 341 auch mit nicht transparenten erfindungsgemäßen Siliconmaterialien ermöglicht wird.

Die hohe Shore D-Härte bzw. das hohe E-Modul ermöglicht eine vorteilhafte Bearbeitung des Bißregistrats mit einer Fräse.

Weiterhin ist aufgrund der hohen Shore D-Härte eine präzise Zuordnung der Ober- und Unterkiefermodelle zueinander gegeben, da das sehr harte Siliconmaterial keine Verschiebung in vertikaler und horizontaler Richtung zuläßt.

Im Gegensatz zu Bißregistriermaterialien mit geringerer Shore-Härte tritt beim erfindungsgemäßen Siliconmaterial aufgrund seiner geringen Elastizität (hohes E-Modul) beim Positionieren im Artikulator keine Komprimierbarkeit und kein Nachfedern auf. Auf diese Weise wird eine präzise Registrierung und Übertragung auf das Modell gewährleistet.

Weiterhin zeichnet sich das erfindungsgemäße Siliconmaterial neben einer praxisgerechten Verarbeitbarkeit und einem guten Fließverhalten während der Verarbeitungszeit durch ein schnelles, patientenfreundliches Abbinden im Mund aus (90 sec. Mundverweildauer bei einer Verarbeitungszeit von mindestens 30 sec.).

Aufgrund der Zusammensetzung des erfindungsgemäßen Siliconmaterials wird ein extrem hoher Platin-Gehalt von über 100 ppm, wie es bei der Verwendung von sehr kurzkettigen, inhibierenden vinylhaltigen Siliconölen, die zu einer eingeschränkten Lagerstabilität mit anschließender Beeinträchtigung der Abbindeeigenschaften führen, erforderlich ist, vermieden.

Weiterhin zeigt die erfindungsgemäße Zusammensetzung des Siliconmaterials auf Basis von Organopolysiloxanen mit zwei Vinylgruppen im Molekül und einer Viskosität von 21 bis 99 mPa·s trotz des hohen Vinylgehaltes keine inhibierende Wirkung auf die Vulkanisationsgeschwindigkeit. In Verbindung mit dem erfindungsgemäßen SiH-Vernetzer und Platinkatalysator werden Vulkanisationen mit "snap Effekt" realisiert.

Aufgrund der hohen Vulkanisationsgeschwindigkeit unter Mundbedingungen wird das hohe Risiko des Verwackelns während der Bißregistrierung im Patientenmund minimiert, was letztlich die Präzision der Bißregistrierung erhöht.

Die Organopolysiloxane a) mit zwei Vinylgruppen im Molekül und einer Viskosität zwischen 21 bis 99 mPa·s umfassen Substanzen der allgemeinen Formel:

$$CH_2=CH-SiR_2O-(SiR_2O)_n-SiR_2-CH=CH_2,$$

worin

R =  Alkyl- (z. B. Methyl-, Ethyl-, Isopropyl-), Aryl- (z. B. Phenyl-, Naphtyl-, Tolyl-, Xylyl), Aralkyl- (Benzyl-, Phenylethyl-) und halogensubstituierte Alkyl- und Aryl-Gruppen (z. B. 3.3.3.-Trifluorpropyl-, Chlorphenyl-, Difluorphenyl-), Cyanalkyl-, Cycloalkyl- und Cycloalkenyl-. Vorzugsweise ist R = Methyl-.

n =  eine ganze Zahl im Wert von 21 bis 69, wobei "n" theoretisch berechnete Werte darstellt, die aus dem experimentell bestimmten Vinylgehalt berechnet wurden, unter der Annahme, daß pro Molekül zwei Vinylgruppen enthalten sind und R = Methyl ist.

Die Organohydrogensiloxan-Komponenten b) fungieren als Vernetzer und sind Polyalkyl-, Polyaryl- und Polyalkylaryl- sowie Polyhalogenalkyl-, Polyhalogenaryl- und Polyhalogenalkylaryl-Siloxane, welche im Molekül mindestens zwei an Siliciumatome gebundene Wasserstoff- ' atome aufweisen.

Verwendet werden Vernetzer mit einem SiH-Gehalt von 1 bis 15 mmol/g.

Als Katalysatoren c) für die Hydrosilylierung sind Salze, Komplexe und kolloidal vorliegende Formen der Übergangsmetalle der 8. Nebengruppe geeignet; vorzugsweise die Metalle Platin, Palladium und Rhodium; insbesondere erweisen sich Platinkomplexe, die z. B. aus Hexachloroplatinsäure bzw. aus Platinsalzen hergestellt werden, als geeignet.

Die Komponente d) ist ein hochdisperser, aktiver Füllstoff mit einer BET-Oberfläche von mindestens 50 m$^2$/g wie Titandioxid, Aluminiumoxid, Zinkoxid und bevorzugterweise naßgefällte oder pyrogen gewonnene Kieselsäure. Die genannten Stoffe können hydrophil oder hydrophobiert vorliegen.

Weiterhin können als verstärkende Füllstoffe faser- oder blättchenförmige Füllstoffe eingesetzt werden, wobei mineralische, faserförmige Füllstoffe, wie z. B. Wollastonit und synthetische, faserförmige Füllstoffe, wie z. B. Glasfasern, Keramikfasern oder Kunststoffasern zum Einsatz kommen können.

Als Füllstoffe e) werden eingesetzt: Metalloxide, Metallhydroxide, Metalloxidhydroxide, Mischoxide und Mischhydroxide, vorzugsweise Siliciumdioxid, insbesondere in Form von Quarz und seinen kristallinen Modifikationen, Quarzgut sowie Aluminiumoxid, Calciumoxid, Aluminiumhydroxid. Auch Füllstoffe wie Calciumcarbonat, Kieselgur, Diatomenerde, Talkum, Glas und Füllstoffe auf Kunststoffbasis, beispielsweise Polymethylmethacrylat, Polycarbonat, Polyvinylchlorid, Siliconharzpulver, Pulver auf Basis fluororganischer Verbindungen sowie organische und anorganische Hohlkugeln, Massivkugeln und Fasern sind geeignet. Desweiteren können volle oder hohle Kunststoffteilchen, z. B. auch in sphärischer Form, auf deren Oberfläche anorganische Füllstoffpartikel eingebettet sind, eingesetzt werden.

Die unter d) und e) genannten Füllstoffe können auch oberflächenbehandelt (gecoated) vorliegen. Die Oberflächenbehandlung kann z. B. mit Silanen und Fettsäuren erfolgen, die funktionelle Gruppen (z. B. Vinyl-, Allyl-, -SiH) aufweisen können.

Bei den unter f) genannten Farbstoffen handelt es sich um lösliche Farbstoffe oder um Pigmentfarbstoffe. Im Bereich der zahnmedizinischen und medizinischen Anwendungen der Silicionmasse werden vorzugsweise Lebensmittelfarbstoffe eingesetzt. Zur Verwendung gelangen gleichfalls Farbpasten aus Polysiloxan- bzw. Mineralöl-Farbstoff-Formulierungen.

Als Feuchtigkeitsbinder g) können Zeolithe, wasserfreies Aluminiumsulfat, Molsieb, Kieselgur und Blaugel eingesetzt werden.

Bei den unter h) genannten Organopolysiloxanen mit mehr als zwei Vinylgruppen im Molekül handelt es sich um vinylendgestoppte und vinylseitenständige Organopolysiloxane mit einer Viskosität von 20 bis 350.000 mPa·s.

Als Inhibitoren i) zur Reaktivitätseinstellung der Hydrosilylierungsreaktion werden kurzkettige Organopolysiloxane der allgemeinen Formel

$$CH_2=CH-SiR_2O-(SiR_2O)_n-SiR_2-CH=CH_2$$

eingesetzt, worin

R gleiche oder verschiedene, ggf. substituierte Kohlenwasserstoffreste, wie z. B. Alkyl-, Alkenyl-, Alkinyl und n = 0 oder eine ganze Zahl von 1 bis 6 bedeutet.
R kann weiterhin ein Alkenyl- oder Alkinyl-terminierter Siloxanrest sein.

Ferner können zum Regeln der Vernetzungsgeschwindigkeit vinylhaltige, cyclische Siloxane, wie beispielsweise Tetra-Vinyltetramethylcyclotetrasiloxan oder endständige Doppel- oder Dreifachbindungen enthaltende, organische Hydroxyl-Verbindungen eingesetzt werden.

Die unter j) genannten vinyl- und ethoxygruppenhaltigen, festen oder flüssigen MQ-Harze sind dadurch gekennzeichnet, daß sie als Q-Einheit das tetrafunktionelle $SiO_{4/2}$ und als M-Bausteine das monofunktionelle $R_3SiO_{1/2}$, wobei R = Vinyl-, Methyl-, Ethyl-, Phenyl- sein kann, enthalten.

Darüber hinaus können auch noch als T-Einheiten das trifunktionelle $RSiO_{3/2}$ und als D-Einheiten das bifunktionelle $R_2SiO_{2/2}$ mit der gleichen Bedeutung für R wie oben vorhanden sein.

Diese MQ-Harze können in Organopolysiloxanen mit zwei oder mehr Vinylgruppen im Molekül und einer Viskosität von 21 bis 350.000 MPa·s gelöst vorliegen.

Der Vinylgruppengehalt der genannten MQ-Harze liegt im Bereich von 0,1 bis 8mmol/g und der Ethoxygruppengehalt liegt bei kleiner 4 mmol/g.

Der SiOH-Gehalt der MQ-Harze liegt so niedrig, daß kein Gasen durch Wasserstoffentwicklung auftritt.

Weiterhin ist der Anteil der flüchtigen Bestandteile der MQ-Harze so gering, daß die Dimensionsstabilität nicht beeinträchtigt wird.

Die erfindungsgemäß eingesetzten Compounds k) setzen sich aus Organopolysiloxanen mit zwei oder mehr Vinylgruppen im Molekül und einer Viskosität von 21 bis 350.000 MPa·s und den unter d) genannten verstärkenden Füllstoffen zusammen. Diese werden unter Verwendung von Modifizierhilfsmitteln, z. B. Hexamethyldisalazan in situ hydrophobiert.

Bei den als Tenside, Emulgatoren und Stabilisatoren eingesetzten Komponenten l) handelt es sich um anionische Tenside, insbesondere Alkylsulfate, Alkylbenzolsulfonate und -phosphate, kationische Tenside, insbesondere Tetraalkylammoniumhalogenide, nichtionische Tenside, insbesondere Alkyl- und Alkylphenyl-Polyalkylalkylenoxide und deren Alkylether und Alkylester, Fettsäurealkylolamide, Saccharosefettsäureester, Trialkylaminoxide, Silicontenside oder Fluortenside sowie amphotere Tenside, insbesondere sulfatierte oder oxyethylierte Kondensationsprodukte aus Alkylenphenolen und Formaldehyd, Ethylenoxid-Propylenoxid-Blockpolymerisate und modifizierte Polysiloxane.

Die Tenside können auch funktionelle Gruppen, wie z. B.-OH-, -CH=CH$_2$, -OCO-(CH$_3$)C=CH$_2$ und SiH enthalten.

Bei den unter m) genannten radioopaken Substanzen handelt es sich um barium-, strontium-, lanthan- oder zinkhaltige Gläser, Bariumsulfat, Zirkondioxid, Lanthanoxid oder um keramische Füllstoffzusammensetzungen, die Oxide von Lanthan, Hafnium und Seltenerdmetallen enthalten.

Weiterhin können komplexe Schwermetallfluoride der allgemeinen Formel M$^{II}$M$^{IV}$F$_6$ oder YF$_3$ eingesetzt werden, wobei M$^{II}$ ein Calcium-, Strontium- oder Bariumion und M$^{IV}$ ein Titan-, Zirkon-, oder Hafniumion bedeutet.

Ferner am Siliconpolymer gebundene Atome bzw. Atomgruppen, die radioopake Eigenschaften besitzen, wie z. B. an Silicium gebundenes Jod.

Bei den unter n) genannten H$_2$-Absorber/Adsorber handelt es sich um feinverteiltes Palladium oder Platin bzw. deren Legierungen, die ggf. in Alumosilikaten enthalten sind.

Weiterhin sind auch Substanzen einsetzbar, die die H$_2$-Entwicklung eliminieren bzw. reduzieren, wie z. B. 3-Methyl-1-butin-3-ol und CH$_3$Si[O-C(CH$_3$)$_2$-C≡CH]$_3$.

Vorzugsweise enthält das erfindungsgemäße Siliconmaterial, d. h. das Gemisch aus beiden Komponenten des additionsvernetzenden 2-Komponenten-Systems, folgende Mengen der einzelnen Komponenten a) bis n) in Gew. %, bezogen auf das gesamte Siliconmaterial:

a) 1 bis 90 Gew.%, vorzugsweise 10 bis 60 Gew.% Organopolysiloxane mit zwei Vinylgruppen im Molekül und einer Viskosität von 21 bis 99 mPa·s

b) 1 bis 40 Gew.%, vorzugsweise 1 bis 20 Gew.% Organohydrogenpolysiloxane

c) 0,0001 bis 0,1 Gew.%, vorzugsweise 0,0005 bis 0,1 Gew.% der Katalysatoren zur Beschleunigung der Hydrosilylierungsreaktion, bezogen auf reines Metall

d) bis 80 Gew.%, vorzugsweise bis 50 Gew.% der verstärkenden Füllstoffe

e) bis 90 Gew.%, vorzugsweise 20 bis 80 Gew.% der nicht verstärkenden Füllstoffe

f) 0 bis 5 Gew.%, vorzugsweise 0 bis 2 Gew.% der Farbstoffe

g) 0 bis 30 Gew.%, vorzugsweise 0 bis 5 Gew.% der Feuchtigkeitsbinder

h) 0 bis 70 Gew.%, vorzugsweise 0 bis 20 Gew.% der Organopolysiloxane mit mehr als zwei Vinylgruppen pro Molekül

i) 0 bis 1,0 Gew.%, vorzugsweise 0 bis 0,1 Gew.% der Inhibitoren

j) 0 bis 90 Gew.%, vorzugsweise 0 bis 50 Gew.% des vinylgruppenhaltigen MQ-Harzes

k) 0 bis 80 Gew.%, vorzugsweise 0 bis 50 Gew.% des Compounds aus vinylgruppenhaltigen Organopolysiloxanen und verstärkenden Füllstoffen

l) 0 bis 10 Gew.%, vorzugsweise 0 bis 5 Gew.% der Tenside, Emulgatoren und Stabilisatoren

m) 0 bis 90 Gew.%, vorzugsweise 0 bis 80 Gew.% der radioopaken Substanzen

n) 0 bis 20 Gew.%, vorzugsweise 0 bis 10 Gew.% der H$_2$-Absorber/Adsorber oder der die H$_2$-Entwicklung reduzierenden bzw. eliminierenden Substanzen.

Nachstehend wird die Erfindung anhand von Beispielen näher erläutert, in denen alle Teile Gewichtsteile bedeuten:

Beispiel 1

In einem geschlossenen Kneter werden 125 Teile eines vinylendgestoppten Polydimethylsiloxans mit einer Viskosität von 50 MPa·s bei 20 °C mit 330 Teilen Aluminiumhydroxid mit einer mittleren Korngröße von 10 μm, 15 Teilen einer pyrogen hergestellten, hochdispersen, hydrophobierten Kieselsäure mit einer Oberfläche von 200 m$^2$/g nach BET, 2 Teilen eines Platin-Katalysators mit einem Gehalt an reinem Platin von 1 %, für 1,5 Stunden homogenisiert und danach 15 Minuten im Vakuum entgast.

Man erhält eine mittelfließende Paste (DIN EN 24823). Die Paste stellt die Komponente A des erfindungsgemäßen 2-Komponenten-Siliconmaterials dar. Nach einer Lagerung bei 23 °C über 1 Monat waren die Viskosität und die Reaktivität im Sollbereich.

Beispiel 2

In einem geschlossenen Kneter werden 100 Teile eines vinylendgestoppten Polydimethylsiloxans, mit einer Viskosität von 50 MPa·s bei 20 °C, mit 40 Teilen eines Polymethylhydrogensiloxans, mit einer Viskosität von 30 MPa·s und einem SiH-Gehalt von 6,9 mmol/g, 350 Teilen Aluminiumhydroxid, 15 Teilen einer pyrogen hergestellten, hochdispersen, hydrophobierten Kieselsäure, mit einer Oberfläche von 200 m$^2$/g, nach BET für 1,5 Stunden homogenisiert und danach 15 Minuten im Vakuum entgast.

Man erhält eine mittelfließende Paste (DIN EN 24823). Die Paste stellt die Komponente B des erfindungsgemäßen 2-Komponenten-Siliconmaterials dar. Nach einer Lagerung bei 23 °C über 1 Monat waren die Viskosität und die Reaktivität im Sollbereich.

Beispiel 3

50 Teile der in Beispiel 1 beschriebenen Komponente A und 50 Teile der in Beispiel 2 beschriebenen Komponente B werden aus einer Kartusche ausgedrückt und über einen statischen Mischer homogen gemischt.
Das Produkt bleibt ca. 30 Sekunden bei Raumtemperatur verarbeitbar und bindet bei einer Temperatur von 35 °C innerhalb von ca. 2 Minuten nach Mischbeginn vollständig ab.
Man erhält als Vulkanisat harte, schwer komprimierbare Formkörper, die sich gezielt abbrechen lassen und gut schneid- und fräsbar sind. Mit dem Shore D-Durometer 3100 der Firma Zwick erhält man eine Shore D-Härte = 45.
Der Elastizitätsmodul im Druckversuch in Anlehnung an DIN 53457 beträgt 36,8 MPa und im Zugversuch (DIN 53455) 45,0 MPa.

Probekörper:     Länge 50 mm, Durchmesser 10 mm, Meßlänge $l_0$ 15 mm, Probekörperform: Zylinder.

Der E-Modul wird als Sekantenmodul zwischen 0,1 und 0,8 % Dehnung/Stauchung nach folgender Gleichung berechnet:

$$E = [R(0,8 \text{ \%}) - R (0,1 \text{ \%})]/(0,8 \text{ \%} - 0,1 \text{ \%}).$$

Beispiel 4

In einem Kneter werden 125 Teile eines vinylendgestoppten Polydimethylsiloxans mit einer Viskosität von 25 MPa·s bei 20 °C, mit 330 Teilen Aluminiumhydroxid mit einer mittleren Korngröße von 10μm, 17 Teilen einer pyrogen hergestellten, hochdispersen, hydrophobierten Kieselsäure mit einer Oberfläche von 200 $m^2$/g nach BET, 3 Teilen eines Platin-Katalysators mit einem Gehalt an reinem Platin von 1 %, für 1,5 Stunden homogenisiert und danach 15 Minuten im Vakuum entgast.
Man erhält eine mittelfließende Paste (DIN EN 24823). Die Paste stellt die Komponente A des erfindungsgemäßen 2-Komponenten-Siliconmaterials dar. Nach einer Lagerung bei 23 °C über 1 Monat waren die Viskosität und die Reaktivität im Sollbereich.

Beispiel 5

In einem Kneter werden 100 Teile eines vinylendgestoppten Polydimethylsiloxans, mit einer Viskosität von 25 MPa·s bei 20 °C, mit 45 Teilen eines Polymethylhydrogensiloxans, mit einer Viskosität von 30 MPa·s und einem SiH-Gehalt von 6,9 mmol/g, 350 Teilen Aluminiumhydroxid, 17 Teilen einer pyrogen hergestellten, hochdispersen, hydrophobierten Kieselsäure mit einer Oberfläche von 200 $m^2$/g nach BET für 1,5 Stunden homogenisiert und danach 15 Minuten im Vakuum entgast.
Man erhält eine mittelfließende Paste (DIN EN 24823). Die Paste stellt die Komponente B des erfindungsgemäßen 2-Komponenten-Siliconmaterials dar. Nach einer Lagerung bei 23 °C über 1 Monat waren die Viskosität und die Reaktivität im Sollbereich.

Beispiel 6

50 Teile der in Beispiel 4 beschriebenen Komponente A und 50 Teile der in Beispiel 5 beschriebenen Komponente B werden aus einer Kartusche ausgedrückt und über einen statischen Mischer homogen gemischt.
Das Produkt bleibt ca. 30 Sekunden bei Raumtemperatur verarbeitbar und bindet bei einer Temperatur von 35 °C innerhalb von ca. 2 Minuten nach Mischbeginn vollständig ab.
Man erhält als Vulkanisat harte, schwer komprimierbare Formkörper, die sich gezielt abbrechen lassen und gut schneid- und fräsbar sind. Mit dem Shore D-Durometer 3100 der Firma Zwick erhält man eine Shore D-Härte = 53.
Der Elastizitätsmodul im Druckversuch, in Anlehnung an DIN 53457 beträgt 63,5 MPa und im Zugversuch (DIN 53455) 69,4 MPa.

Probekörper:     Länge 50 mm, Durchmesser 10 mm, Meßlänge $l_0$ 15 mm, Probekörperform: Zylinder.

Der E-Modul wird als Sekantenmodul zwischen 0,1 und 0,8 % Dehnung/Stauchung nach folgender Gleichung berechnet:

$$E = [R(0,8 \text{ \%}) - R (0,1 \text{ \%})]/(0,8 \text{ \%} - 0,1 \text{ \%}).$$

Beispiel 7 (Vergleichsbeispiel)

In einem Kneter werden 140 Teile eines vinylendgestoppten Polydimethylsiloxans, mit einer Viskosität von 1.000 MPa·s bei 20 °C, mit 10 Teilen eines vinylgruppenhaltigen MQ-Harzes, gelöst in einem vinylendgestoppten Polydimethylsiloxan mit einer Gesamtviskosität von 6.000 MPa·s, 10 Teilen einer pyrogen hergestellten, hochdispersen, hydrophobierten Kieselsäure mit einer Oberfläche von 200 m$^2$/g nach BET, 350 Teilen Aluminiumhydroxid, 1,5 Teilen eines Platin-Katalysators mit einem Gehalt an reinem Platin von 1 %, für 1,5 Stunden homogenisiert und danach 15 Minuten im Vakuum entgast.

Man erhält eine mittelfließende Paste (DIN EN 24823). Die Paste stellt die Komponente A eines nicht erfindungsgemäßen 2-Komponenten-Siliconmaterials dar. Nach einer Lagerung bei 23 °C über 1 Monat waren die Viskosität und die Reaktivität im Sollbereich.

Beispiel 8 (Vergleichsbeispiel)

In einem Kneter werden 100 Teile eines vinylendgestoppten Polydimethylsiloxans, mit einer Viskosität von 1.000 MPa·s bei 20 °C, mit 10 Teilen eines vinylgruppenhaltigen MQ-Harzes, gelöst in einem vinylendgestoppten Polydimethylsiloxan mit einer Gesamtviskosität von 6.000 MPa·s, 40 Teilen eines Polymethylhydrogensiloxans, mit einer Viskosität von 50 MPa·s und einem SiH-Gehalt von 2,3 mmol/g, 350 Teilen Aluminiumhydroxid, 12 Teilen einer pyrogen hergestellten, hochdispersen, hydrophobierten Kieselsäure mit einer Oberfläche von 200 m$^2$/g nach BET, für 1,5 Stunden homogenisiert und danach 15 Minuten im Vakuum entgast.

Man erhält eine mittelfließende Paste (DIN EN 24823). Die Paste stellt die Komponente B eines nicht erfindungsgemäßen 2-Komponenten-Siliconmaterials dar. Nach einer Lagerung bei 23 °C über 1 Monat waren die Viskosität und die Reaktivität im Sollbereich.

Beispiel 9 (Vergleichsbeispiel)

50 Teile der ein Beispiel 7 beschriebenen Komponente A und 50 Teile der in Beispiel 8 beschriebenen Komponente B werden aus einer Kartusche ausgedrückt und über einen statischen Mischer homogen gemischt.

Die als Vulkanisat erhaltenen Formkörper sind mittelhart und elastisch verformbar. Eine Shore D-Härte ist nicht sinnvoll meßbar. Die Shore A-Härte, gemessen mit dem Shore A-Durometer der Firma Zwick, beträgt Shore A = 70.

Der Elastizitätsmodul im Druckversuch, in Anlehnung an DIN 53457 beträgt 4,4 MPa und im Zugversuch (DIN 53455) 5,0 MPa.

Probekörper:      Länge 50 mm, Durchmesser 10 mm, Meßlänge $l_0$ 15 mm, Probekörperform: Zylinder.

Der E-Modul wird als Sekantenmodul zwischen 0,1 und 0,8 % Dehnung/Stauchung nach folgender Gleichung berechnet:

$$E = [R(0,8 \text{ \%}) - R (0,1 \text{ \%})]/(0,8 \text{ \%} - 0,1 \text{ \%}).$$

Diese Beispiele verdeutlichen, daß die erfindungsgemäße Zusammensetzung des Siliconmaterials sich entscheidend auf Härte und Elastizitätsmodul der resultierenden Vulkanisate auswirkt, wobei eine extrem schnelle Vulkanisation bei praxisgerechter Verarbeitungszeit realisiert wird, d. h. die erfindungsgemäßen Organopolysiloxane mit zwei Vinylgruppen im Molekül und einer Viskosität von 21 bis 99 mPa·s zeigen keine inhibierende Wirkung, wie dies den Tabellen 1 und 2 zu entnehmen ist.

Beispiel 10 (Vergleichsbeispiel)

Ein handelsübliches Bißregistrierungsmaterial auf Basis additionsvernetzender Silicone wird gemäß Anweisung des Herstellers gemischt und zur Abbindung gebracht.

Die mit dem Shore A-Durometer der Firma Zwick gemessene Shore A-Härte beträgt Shore A = 85, die Shore D-Härte beträgt Shore D = 28.

Der Elastizitätsmodul im Druckversuch in Anlehnung an DIN 53457 beträgt 13,1 MPa und im Zugversuch (DIN 53455) 16,9 MPa.

Dieses Beispiel soll verdeutlichen, daß Bißregistriermaterialien auf Basis additionsvernetzender Silicone nach dem Stand der Technik über eine merklich geringere Härte bzw. Elastizitätsmoduli verfügen.

Beispiel 11 (Vergleichsbeispiel)

Ein transparentes Bißregistriermaterial (lt. Herstellerangabe extra hart) auf Basis additionsvernetzender Silicone wird gemäß Anweisung des Herstellers gemischt und zur Abbindung gebracht.

Die mit dem Shore A-Durometer der Firma Zwick gemessene Shore A-Härte beträgt Shore A = 78, die Shore D-Härte beträgt Shore D = 19.

Der E-Modul im Druckversuch beträgt 8,2 MPa (DIN 53457). Der E-Modul im Zugversuch beträgt 8,6 MPa (DIN 53455).

## Tabelle 1

| Beispiel Nr. | Shore A | Shore D | Elastizitätsmodul | |
| --- | --- | --- | --- | --- |
| | | | Druckversuch | Zugversuch |
| 3 | > 95 | 45 | 36,8 MPa | 45,0 MPa |
| 6 | > 95 | 53 | 63,5 MPa | 69,4 MPa |
| 9 | 70 | -[*1] | 4,4 MPa | 5,0 MPa |
| 10 | 85 | 28 | 13,1 MPa | 16,9 MPa |
| 11[*2] | 78 | 19 | 8,2 Mpa | 8,6 MPa |

*1 = nicht sinnvoll meßbar
*2 = transparentes Verkaufsprodukt (laut Herstellerangabe extra hart), daß aus dem Patent EP-A-O 522 341 hervorgeht

## Tabelle 2

| Beispiel Nr. | Austragkraft aus der Kartusche | Verarbeitungszeit | Mundverweildauer | gezielte Brechbarkeit | Fräsbarkeit |
| --- | --- | --- | --- | --- | --- |
| 3 | gering | ≥ 30sec | 90 sec | sehr gut | sehr gut |
| 6 | gering | ≥ 30 sec | 90 sec | sehr gut | sehr gut |
| 9 | hoch | 60 sec | 180 sec | nicht möglich | nur bedingt |
| 10 | mittel | ≤ 15 sec | 90 sec | möglich | möglich |
| 11[*2] | mittel | ≥ 30 sec | 210 sec | nicht möglich | nur bedingt |

EP 0 894 117 B1

**Patentansprüche**

1.  Additionsvernetzendes 2-Komponenten-Siliconmaterial enthaltend:

    a) Organopolysiloxane mit Vinylgruppen im Molekül,
    b) als Vernetzer Organohydrogenpolysiloxane mit zwei oder mehr SiH-Gruppen und einem SiH-Gehalt von 1-15 mmol/g
    c) als Katalysator Übergangsmetalle und Übergangsmetallverbindungen der 8. Nebengruppe
    d) verstärkende Füllstoffe mit einer BET-Oberfläche von mindestens 50 $m^2$/g
    e) nicht verstärkende Füllstoffe

    dadurch gekennzeichnet, dass der Inhaltsstoff a) ein Organopolysiloxan mit zwei Vinylgruppen im Molekül mit einer Viskosität zwischen 21 und 99 mPa·s (20°C) ist und das Siliconmaterial eine Shore D-Härte von größer 35 und ein Elastizitätsmodul von größer 20 MPa (gemessen nach DIN 53457 oder 53455) im ausvulkanisierten Zustand aufweist.

2.  Siliconmaterial nach Anspruch 1, dadurch gekennzeichnet, dass die verstärkenden Füllstoffe nach Komponente d) hochdisperse, aktive Füllstoffe mit einer BET-Oberfläche von mindestens 50 $m^2$/g wie Titandioxid, Aluminiumoxid, Zinkoxid, naßgefällte oder pyrogen gewonnene Kieselsäure ist, wobei die genannten Stoffe hydrophil oder hydrophobiert vorliegen, oder verstärkende faser- oder blättchenförmige Füllstoffe, mineralische, faserförmige Füllstoffe, wie Wollastonit, und synthetische, faserförmige Füllstoffe, wie Glasfasern, Keramikfasern oder Kunststofffasern enthält.

3.  Siliconmaterial nach Anspruch 1, dadurch gekennzeichnet, dass die nicht verstärkenden Füllstoffe nach Komponente e) Metalloxide, Metallhydroxide, Metalloxidhydroxide, Mischoxide und Mischhydroxide, vorzugsweise Siliciumdioxid, auch in Form von Quarz, und seinen kristallinen Modifikationen, Quarzgut, Aluminiumoxid, Calciumoxid, Aluminiumhydroxid, Calciumcarbonat, Kieselgur, Diatomenerde, Talkum, Glas, Füllstoffe auf Kunststoffbasis, Polymethylmethacrylat, Polycarbonat, Polyvinylchlorid, Siliconharzpulver, Pulver auf Basis fluororganischer Verbindungen sowie organische und anorganische Hohlkugeln, Massivkugeln und Fasern, sowie volle oder hohle Kunststoffteilchen, auch in sphärischer Form, auf deren Oberfläche anorganische Füllstoffartikel eingebettet sind.

4.  Siliconmaterial nach einem der Ansprüche 1 bis 3, dadurch gekennzeichnet, dass das Siliconmaterial
    f) Farbstoffe
    enthält.

5.  Siliconmaterial nach einem der Ansprüche 1 bis 4, dadurch gekennzeichnet, dass das Siliconmaterial
    g) Feuchtigkeitsbinder
    enthält.

6.  Siliconmaterial nach einem der Ansprüche 1 bis 5, dadurch gekennzeichnet, dass das Siliconmaterial
    h) Organopolysiloxane mit mehr als zwei Vinylgruppen im Molekül
    enthält.

7.  Siliconmaterial nach einem der Ansprüche 1 bis 6, dadurch gekennzeichnet, dass das Siliconmaterial
    i) Inhibitoren zur Reaktivitätseinstellung
    enthält.

8.  Siliconmaterial nach einem der Ansprüche 1 bis 7, dadurch gekennzeichnet, dass das Siliconmaterial
    j) vinylgruppenhaltige, feste oder flüssige MQ-Harze
    enthält.

9.  Siliconmaterial nach einem der Ansprüche 1 bis 8, dadurch gekennzeichnet, dass das Siliconmaterial
    k) Compounds aus Organopolysiloxanen und verstärkenden Füllstoffen
    enthält.

10. Siliconmaterial nach einem der Ansprüche 1 bis 9, dadurch gekennzeichnet, dass das Siliconmaterial
    l) Tenside, Emulgatoren und Stabilisatoren
    enthält.

**11.** Siliconmaterial nach einem der Ansprüche 1 bis 10, dadurch gekennzeichnet, dass das Siliconmaterial
m) radioopake Substanzen
enthält.

**12.** Siliconmaterial nach einem der Ansprüche 1 bis 11, dadurch gekennzeichnet, dass das Siliconmaterial
n) $H_2$-absorbierende oder adsorbierende Substanzen und Substanzen, die die $H_2$-Entwicklung eliminieren oder reduzieren,
enthält.

**13.** Siliconmaterial nach einem der Anspruche 1 bis 12, dadurch gekennzeichnet, dass es Organopolysiloxane (a) mit zwei Vinylgruppen im Molekül und einer Viskosität zwischen 21 bis 99 mPa·s (20°C) umfassend Substanzen der allgemeinen Formel:

$$CH_2=CH-SiR_2O-(SiR_2O)_n-SiR_2-CH=CH_2,$$

worin

R = Alkyl- (z.B. Methyl-, Ethyl-, Isopropyl-), Aryl- (z.B. Phenyl-, Naphtyl-, Tolyl-, Xylyl), Aralkyl- (Benzyl-, Phenylethyl-) und halogensubstituierte Alkyl- und Aryl-Gruppen (z.B. 3,3,3.-Trifluorpropyl-, Chlorphenyl-, Difluorphenyl-), Cyanalkyl-, Cycloalkyl- und Cycloalkenyl-,
vorzugsweise R = Methyl ist
n = eine ganze Zahl im Wert von 21 bis 69, wobei "n" theoretisch berechnete Werte darstellt, die aus dem experimentell bestimmten Vinylgehalt berechnet wurden, unter der Annahme, dass pro Molekül zwei Vinylgruppen enthalten sind und
R = Methyl ist,

enthalten.

**14.** Siliconmaterial nach einem der Ansprüche 1 bis 13, dadurch gekennzeichnet, dass die Organohydrogenpolysiloxan-Komponenten (b) als Vernetzer Polyalkyl-, Polyaryl- und Polyalkylaryl- sowie Polyhalogenalkyl-, Polyhalogenaryl- und Polyhalogenalkylaryl-Siloxane sind, welche im Molekül mindestens zwei an Siliciumatome gebundene Wasserstoffatome aufweisen, wobei Vernetzer mit einem SiH-Gehalt von 1 bis 15 mmol/g verwendet werden.

**15.** Siliconmaterial nach einem der Ansprüche 1 bis 14, dadurch gekennzeichnet, dass als Katalysatoren (c) für die Hydrosilylierung Salze, Komplexe und kolloidal vorliegende Formen der Übergangsmetalle der 8. Nebengruppe, vorzugsweise die Metalle Platin, Palladium und Rhodium, insbesondere Platinkomplexe, die z.B., aus Hexachloroplatinsäure bzw. aus Platinsalzen hergestellt werden, verwendet werden.

**16.** Siliconmaterial nach einem der Ansprüche 1 bis 15, dadurch gekennzeichnet, dass die verstärkenden und nicht verstärkenden Füllstoffe (d) und (e) oberflächenbehandelt (gecoated) vorliegen, wobei die Oberflächenbehandlung, z.B. mit Silanen und Fettsäuren erfolgt, die funktionelle Gruppen, wie Vinyl-, Allyl-, -SiH, aufweisen.

**17.** Siliconmaterial nach einem der Ansprüche 1 bis 16, dadurch gekennzeichnet, dass die Farbstoffe (f) lösliche Farbstoffe oder Pigmentfarbstoffe, Lebensmittelfarbstoffe und Farbpasten aus Polysiloxan- bzw. Mineralöl-Farbstoff-Formulierungen sind.

**18.** Siliconmaterial nach einem der Ansprüche 1 bis 17, dadurch gekennzeichnet, dass als Feuchtigkeitsbinder (g) Zeolithe, wasserfreies Aluminiumsulfat, Molsieb, Kieselgur und Blaugel eingesetzt werden.

**19.** Siliconmaterial nach einem der Ansprüche 1 bis 18, dadurch gekennzeichnet, dass die Organopolysiloxane mit mehr als zwei Vinylgruppen im Molekül (h) vinylendgestoppte und vinylseitenständige Organopolysiloxane mit einer Viskosität von 21 bis 350.000 mPa·s (20°C) sind.

**20.** Siliconmaterial nach einem der Ansprüche 1 bis 19, dadurch gekennzeichnet, dass als Inhibitoren (i) zur Reaktivitätseinstellung der Hydrosilylierungsreaktion kurzkettige Organopolysiloxane der allgemeinen Formel

$$CH_2=CH-SiR_2O-(SiR_2O)_n-SiR_2-CH=CH_2$$

eingesetzt werden, worin

R gleiche oder verschiedene, gegebenenfalls substituierte Kohlenwasserstoffreste, wie z.B. Alkyl-, Alkenyl-, Alkinyl und

n = 0 oder eine ganze Zahl von 1 bis 6 bedeutet,

wobei R weiterhin ein alkenyl- oder alkinylterminierter Siloxanrest ist.

21. Siliconmaterial nach Anspruch 20, dadurch gekennzeichnet, dass zum Regeln der Vernetzungsgeschwindigkeit vinylhaltige, cyclische Siloxane, Tetra-Vinyltetramethylcyclotetrasiloxan, endständige Doppel- oder Dreifachbindungen enthaltende, organische Hydroxyl-Verbindungen eingesetzt werden.

22. Siliconmaterial nach einem der Ansprüche 1 bis 21, dadurch gekennzeichnet, dass die Q-Einheiten das tetrafunktionelle $SiO_{4/2}$ und als M-Bausteine das monofunktionelle $R_3SiO_{1/2}$ enthalten.

23. Siliconmaterial nach einem der Ansprüche 1 bis 22, dadurch gekennzeichnet, dass die vinyl- und ethoxygruppenhaltigen, festen oder flüssigen MQ-Harze als T-Einheiten das trifunktionelle $RSiO_{3/2}$ und als D-Einheiten das bifunktionelle $R_2SiO_{2/2}$ enthalten, wobei R Vinyl-, Methyl-, Ethyl-, Phenyl- ist.

24. Siliconmaterial nach Anspruch 23, dadurch gekennzeichnet, dass MQ-Harze in Organopolysiloxanen mit zwei oder mehr Vinylgruppen im Molekül und einer Viskosität von 21 bis 350.000 mPa·s (20°C) gelöst verwendet werden, wobei der Vinylgruppengehalt der MQ-Harze im Bereich von 0,1 bis 8 mmol/g und der Ethoxygruppengehalt bei kleiner 4 mmol/g liegt.

25. Siliconmaterial nach Anspruch 22, dadurch gekennzeichnet, dass der SiOH-Gehalt der MQ-Harze so niedrig liegt, dass kein Gasen durch Wasserstoffentwicklung auftritt, wobei der Anteil der flüchtigen Bestandteile der MQ-Harze so gering ist, dass die Dimensionsstabilität nicht beeinträchtigt wird.

26. Siliconmaterial nach einem der Ansprüche 1 bis 25, dadurch gekennzeichnet, dass die Compounds (k) sich aus Organopolysiloxanen mit zwei oder mehr Vinylgruppen im Molekül und einer Viskosität von 21 bis 350.000 mPa·s (20°C) und den verstärkenden Füllstoffen zusammensetzen, wobei diese unter Verwendung von Modifizierhilfsmitteln, z.B. Hexamethyldisalazan in situ, hydrophobiert werden.

27. Siliconmaterial nach einem der Ansprüche 1 bis 26, dadurch gekennzeichnet, dass es sich bei den als Tenside, Emulgatoren und Stabilisatoren eingesetzten Komponenten (1) um anionische Tenside, insbesondere Alkylsulfate, Alkylbenzolsulfonate und -phosphate, kationische Tenside, Tetraalkylammoniumhalogenide, nichtionische Tenside, insbesondere Alkyl- und Alkylphenyl-Polyalkylalkylenoxide und deren Alkylether und Alkylester, Fettsäurealkylolamide, Saccharosefettsäureester, Trialkylaminoxide, Silicontenside oder Fluortenside sowie amphotere Tenside, sulfatierte oder oxyethylierte Kondensationsprodukte aus Alkylenphenolen und Formaldehyd, Ethylenoxid-Propylenoxid-Blockpolymerisate und modifizierte Polysiloxane, handelt, wobei die Tenside auch funktionelle Gruppen, wie -OH, $-CH=CH_2$, $-OCO-(CH_3)C=CH_2$ und SiH enthalten.

28. Siliconmaterial nach einem der Ansprüche 1 bis 27, dadurch gekennzeichnet, dass als radioopake Substanzen barium-, strontium-, lanthan-oder zinkhaltige Gläser, Bariumsulfat, Zirkondioxid, Lanthanoxid oder keramische Füllstoffzusammensetzungen, die Oxide von Lanthan, Hafnium und Seltenerdmetallen, komplexe Schwermetallfluoride der allgemeinen Formel $M^{II}M^{IV}F_6$ oder $YF_3$, wobei $M^{II}$ ein Calcium-, Strontium-oder Bariumion und $M^{IV}$ ein Titan-, Zirkon-, oder Hafniumion bedeutet, sowie am Siliconpolymer gebundene Atome bzw. Atomgruppen, die radioopake Eigenschaften besitzen, wie an Silicium gebundenes Jod, verwendet werden.

29. Siliconmaterial nach einem der Ansprüche 1 bis 28, dadurch gekennzeichnet, dass es sich bei den unter (n) genannten $H_2$-Absorber/Adsorber um feinverteiltes Palladium oder Platin oder deren Legierungen, die in Alumosilikaten enthalten sind, sowie um Substanzen, die die $H_2$-Entwicklung eliminieren oder reduzieren, wie 3-Methyl-1-butin-3-ol und $CH_3Si[O-C(CH_3)_2-C\equiv CH]_3$, handelt.

**30.** Siliconmaterial nach einem der Ansprüche 1 bis 29, dadurch gekennzeichnet, dass das Siliconmaterial folgende Mengen der einzelnen Komponenten (a) bis (n) in Gew.%, bezogen auf des gesamte Siliconmaterial, enthält:

a) 1 bis 90 Gew.%, vorzugsweise 10 bis 60 Gew.% Organopolysiloxane mit zwei Vinylgruppen im Molekül und einer Viskosität von 21 bis 99 mPa5 (20°C)

b) 1 bis 40 Gew.%, vorzugsweise 1 bis 20 Gew.% Organohydrogenpolysiloxane

c) 0,0001 bis 0,1 Gew.%, vorzugsweise 0,0005 bis 0,1 Gew.% der Katalysatoren zur Beschleunigung der Hydrosilylierungsreaktion, bezogen auf reines Metall

d) bis 80 Gew.%, vorzugsweise bis 50 Gew.% der verstärkenden Füllstoffe

e) bis 90 Gew.%, vorzugsweise 20 bis 80 Gew.% der nicht verstärkenden Füllstoffe

f) 0 bis 5 Gew.%, vorzugsweise 0 bis 2 Gew.% der Farbstoffe

g) 0 bis 30 Gew.%, vorzugsweise 0 bis 5 Gew.% der Feuchtigkeitsbinder

h) 0 bis 70 Gew.%, vorzugsweise 0 bis 20 Gew.% der Organopolysiloxane mit mehr als zwei Vinylgruppen pro Molekül

i) 0 bis 1,0 Gew.%, vorzugsweise 0 bis 0,1 Gew.% der Inhibitoren

j) 0 bis 90 Gew.%, vorzugsweise 0 bis 50 Gew.% des vinylgruppenhaltigen MQ-Harzes

k) 0 bis 80 Gew.%, vorzugsweise 0 bis 50 Gew.% des Compounds aus vinylgruppenhaltigen Organopolysiloxanen und verstärkenden Füllstoffen

l) 0 bis 10 Gew.%, vorzugsweise 0 bis 5 Gew.% der Tenside, Emulgatoren und Stabilisatoren

m) 0 bis 90 Gew.%, vorzugsweise 0 bis 80 Gew.% der radioopaken Substanzen

n) 0 bis 20 Gew.%, vorzugsweise 0 bis 10 Gew.% der $H_2$-Absorber/Adsorber oder der die $H_2$-Entwicklung reduzierenden bzw. eliminierenden Substanzen.

**31.** Verwendung eines additionsvernetzenden 2-Komponenten-Siliconmaterials als spritzbares Material für die Bißregistrierung, Material zur Beschichtung der Bißgabel oder Registrierungsplatten bei der Registrierung, Schlüsselmaterial, z.B. für intraorale Stützstift-Registrate, Übertragung von Brackets in der Kieferorthopädie, Remontage von Keramik-, Guß- oder Kunststofffüllungen zum Einschleifen der Occlusion auf dem Modell, hartwerdendes Unterfütterungssilicon, Modellmaterial zur Herstellung von Inlays/Onlays, mit einer Shore D-Härte von größer 35 und einem Elastizitätsmodul von größer 20 MPa gemessen nach DIN 53457 oder DIN 53455 im ausvulkanisierten Zustand, bestehend aus

a) Organopolysiloxanen mit zwei Vinylgruppen im Molekül und mit einer Viskosität zwischen 21 bis 99 mPa•s (20°C),

b) als Vernetzer Organohydrogenpolysiloxanen mit zwei oder mehr SiH-Gruppen im Molekül und mit einem SiH-Gehalt von 1 bis 15 mmol/g,

c) als Katalysator Übergangsmetalle und Übergangsmetallverbindungen der 8.Nebengruppe zur Beschleunigung der Hydrosilylierungsreaktion

d) verstärkende mit einer BET-Oberfläche von mindestens 50m$^2$/g Füllstoffe und

e) nicht verstärkende Füllstoffe.

**Claims**

1. An addition-cross-linkable two-component silicone material, containing:

   a) organopolysiloxanes having vinyl groups in the molecule;

   b) organohydrogenpolysiloxanes having two or more SiH groups and an SiH content of from 1 to 15 mmol/g as cross-linking agents;

   c) transition metals and transition metal compounds of the 8th subgroup as catalysts;

   d) reinforcing fillers having a BET surface area of at least 50 $m^2$/g;

   e) non-reinforcing fillers;

   characterized in that ingredient a) is an organopolysiloxane having two vinyl groups in the molecule and a viscosity of between 21 and 99 mPa·s (20 °C), and the silicone material has a Shore D hardness of greater than 35 and a modulus of elasticity of greater than 20 MPa (measured according to DIN 53457 or 53455) in the cured state.

2. The silicone material according to claim 1, characterized in that said reinforcing fillers of component d) are highly dispersed active fillers having a BET surface area of at least 50 $m^2$/g, such as titanium dioxide, aluminum oxide, zinc oxide, wet-precipitated or pyrogenic silicic acid, wherein the mentioned materials are in a hydrophilic or hydrophobized form, or reinforcing fibrous or plate-like fillers, mineral fibrous fillers, such as wollastonite, and synthetic fibrous fillers, such as glass gibers, ceramic fibers or plastic fibers.

3. The silicone material according to claim 1, characterized in that said non-reinforcing fillers of component e) are metal oxides, metal hydroxides, metal oxide hydroxides, mixed oxides and mixed hydroxides, preferably silica, also in the form of quartz and its crystalline modifications, fused silica, alumina, calcium oxide, aluminum hydroxide, calcium carbonate, kieselguhr, diatomaceous earth, talcum, glass, plastic-based fillers, polymethyl methacrylate, polycarbonate, polyvinyl chloride, silicone resin powder, powder based on fluoro-organic compounds, and organic and inorganic hollow spheres, beads and fibers, and solid or hollow plastic particles, also in spherical shapes, on the surface of which inorganic filler articles are embedded.

4. The silicone material according to any of claims 1 to 3, characterized by containing
   f) dyes.

5. The silicone material according to any of claims 1 to 4, characterized by containing
   g) moisture-binding agents.

6. The silicone material according to any of claims 1 to 5, characterized by containing
   h) organopolysiloxanes having more than two vinyl groups in the molecule.

7. The silicone material according to any of claims 1 to 6, characterized by containing
   i) inhibitors for reactivity adjustment.

8. The silicone material according to any of claims 1 to 7, characterized by containing
   j) solid or liquid MQ resins containing vinyl groups.

9. The silicone material according to any of claims 1 to 8, characterized by containing
   k) compounds of organopolysiloxanes and reinforcing fillers.

10. The silicone material according to any of claims 1 to 9, characterized by containing
    l) surfactants, emulsifiers and stabilizers.

11. The silicone material according to any of claims 1 to 10, characterized by containing
    m) radiopaque substances.

12. The silicone material according to any of claims 1 to 11, characterized by containing

n) $H_2$-absorbing or $H_2$-adsorbing substances and substances which eliminate or reduce $H_2$ evolution.

13. The silicone material according to any of claims 1 to 12, characterized by containing organopolysiloxanes (a) having two vinyl groups in the molecule and a viscosity of between 21 and 99 mPa·s (20 °C), comprising substances of general formula

$$CH_2=CH-SiR_2O-(SiR_2O)_n-SiR_2-CH=CH_2,$$

wherein

R = alkyl (e.g., methyl, ethyl, isopropyl), aryl (e.g., phenyl, naphthyl, tolyl, xylyl), aralkyl (benzyl, phenylethyl) and halogen-substituted alkyl and aryl groups (e.g., 3,3,3-trifluoropropyl, chlorophenyl, difluorophenyl), cyanoalkyl, cycloalkyl and cycloalkenyl;

preferably, R = methyl;

n = an integer of from 21 to 69, wherein "n" represents theoretically calculated values, calculated from the experimentally determined vinyl content with the supposition that two vinyl groups are contained per molecule and R = methyl.

14. The silicone material according to any of claims 1 to 13, characterized in that said organohydrogenpolysiloxane components (b) used as cross-linking agents are polyalkyl-, polyaryl-, polyalkylaryl-, polyhaloalkyl-, polyhaloaryl and polyhaloalkylaryl siloxanes having at least two hydrogen atoms bonded to silicon atoms in the molecule, wherein cross-linking agents having an SiH content of from 1 to 15 mmol/g are employed.

15. The silicone material according to any of claims 1 to 14, characterized in that salts, complexes and colloidal forms of the transition metals of the 8th subgroup, preferably the metals platinum, palladium and rhodium, especially platinum complexes, for example, those prepared from hexachloroplatinic acid or from platinum salts, are used as said catalysts (c) for hydrosilylation.

16. The silicone material according to any of claims 1 to 15, characterized in that said reinforcing and non-reinforcing fillers (d) and (e) are present in a surface-treated (coated) form, said surface treatment being effected, for example, with silanes and fatty acids having functional groups such as vinyl, allyl, SiH.

17. The silicone material according to any of claims 1 to 16, characterized in that said dyes (f) are soluble dyes or pigment dyes, food colorants and color pastes made of polysiloxane/dye or mineral oil/dye formulations.

18. The silicone material according to any of claims 1 to 17, characterized in that zeolites, anhydrous aluminum sulfate, molecular sieve, kieselguhr and blue gel are employed as said moisture-binding agents (g).

19. The silicone material according to any of claims 1 to 18, characterized in that said organopolysiloxanes having more than two vinyl groups in the molecule (h) are organopolysiloxanes with terminal and/or pendant vinyl groups having a viscosity of from 21 to 350,000 mPa·s (20 °C).

20. The silicone material according to any of claims 1 to 19, characterized in that short-chained organopolysiloxanes of general formula

$$CH_2=CH-SiR_2O-(SiR_2O)_n-SiR_2-CH=CH_2,$$

wherein

R means the same or different hydrocarbon residues which may optionally be substituted, such as alkyl, alkenyl, alkynyl; and

n = 0 or an integer of from 1 to 6;

wherein R may also be an alkenyl-terminated or alkynyl-terminated siloxane residue;

are employed as said inhibitors (i) for reactivity adjustment of the hydrosilylation reaction.

21. The silicone material according to claim 20, characterized in that vinyl-containing cyclic siloxanes, tetravinyltetramethylcyclotetrasiloxane or organic hydroxy compounds containing terminal double or triple bonds are employed for controlling the cross-linking rate.

22. The silicone material according to any of claims 1 to 21, characterized in that said MQ resins contain tetrafunctional $SiO_{4/2}$ as the Q units and monofunctional $R_3SiO_{1/2}$ as the M units.

23. The silicone material according to any of claims 1 to 22, characterized in that said solid or liquid MQ resins containing vinyl and ethoxy groups contain trifunctional $RSiO_{3/2}$ as the T units and bifunctional $R_2SiO_{2/2}$ as the D units, R being vinyl, methyl, ethyl, phenyl.

24. The silicone material according to claim 23, characterized in that MQ resins are used in a form dissolved in organopolysiloxanes having two or more vinyl groups in the molecule and a viscosity of from 21 to 350,000 mPa·s (20 °C), the vinyl group content of said MQ resins being within a range of from 0.1 to 8 mmol/g and their ethoxy group content being lower than 4 mmol/g.

25. The silicone material according to claim 22, characterized in that the SiOH content of the MQ resins is so low that gassing due to hydrogen evolution does not occur, wherein the volatiles content of the MQ resins is so low that dimensional stability is not adversely affected.

26. The silicone material according to any of claims 1 to 25, characterized in that said compounds (k) are composed of organopolysiloxanes having two or more vinyl groups in the molecule and a viscosity of from 21 to 350,000 mPa·s (20 °C) and the reinforcing fillers, the fillers being hydrophobized using modification aids, e.g., hexamethyldisilazane in situ.

27. The silicone material according to any of claims 1 to 26, characterized in that the components (I) being employed as surfactants, emulsifiers and stabilizers are anionic surfactants, especially alkyl sulfates, alkylbenzenesulfonates and -phosphates, cationic surfactants, tetraalkylammonium halides, non-ionic surfactants, especially alkyl and alkylphenyl polyalkyl alkylene oxides and their alkyl ethers and alkyl esters, fatty acid alkylol amides, saccharose fatty acid esters, trialkylamine oxides, silicone surfactants or fluorosurfactants, and amphoteric surfactants, sulfated or oxyethylated condensation products of alkylene phenols and formaldehyde, ethylene oxide/propylene oxide block polymers and modified polysiloxanes, wherein said surfactants also contain functional groups such as -OH, -CH=CH$_2$, -OCO-(CH$_3$)C=CH$_2$ and SiH.

28. The silicone material according to any of claims 1 to 27, characterized in that barium-, strontium-, lanthanum- or zinc-containing glasses, barium sulfate, zirconium dioxide, lanthanum oxide or ceramic filler compositions, the oxides of lanthanum, hafnium and rare earth metals, complex heavy metal fluorides of general formula $M^{II}M^{IV}F_6$ or $YF_3$, wherein $M^{II}$ is a calcium, strontium or barium ion and $M^{IV}$ is a titanium, zirconium or hafnium ion, and atoms or atom groups which have radiopaque properties and are bound to the silicone polymer, such as iodine bonded to silicon, are used as said radiopaque substances.

29. The silicone material according to any of claims 1 to 28, characterized in that the H$_2$ absorbers/adsorbers mentioned under (n) are finely divided palladium or platinum or their alloys contained in alumosilicates, or substances which eliminate or reduce H$_2$ evolution, such as 3-methyl-1-butyne-3-ol and CH$_3$Si[O-C(CH$_3$)$_2$-C≡CH]$_3$.

30. The silicone material according to any of claims 1 to 29, characterized by containing the following amounts of the individual components (a) to (n) in % by weight, based on the total silicone material:

   a) from 1 to 90% by weight, preferably from 10 to 60% by weight, of organopolysiloxanes having two vinyl groups in the molecule and a viscosity of from 21 to 99 mPa·s (20 °C);

   b) from 1 to 40% by weight, preferably from 1 to 20% by weight, of organohydrogenpolysiloxanes;

   c) from 0.0001 to 0.1% by weight, preferably from 0.0005 to 0.1% by weight, of said catalysts for accelerating the hydrosilylation reaction, based on the pure metal;

d) up to 80% by weight, preferably up to 50% by weight, of said reinforcing fillers;

e) up to 90% by weight, preferably from 20 to 80% by weight, of said non-reinforcing fillers;

f) from 0 to 5% by weight, preferably from 0 to 2% by weight, of said dyes;

g) from 0 to 30% by weight, preferably from 0 to 5% by weight, of said moisture-binding agents;

h) from 0 to 70% by weight, preferably from 0 to 20% by weight, of said organopolysiloxanes having more than two vinyl groups per molecule;

i) from 0 to 1.0% by weight, preferably from 0 to 0.1% by weight, of said inhibitors;

j) from 0 to 90% by weight, preferably from 0 to 50% by weight, of said MQ resin containing vinyl groups;

k) from 0 to 80% by weight, preferably from 0 to 50% by weight, of said compound of vinyl-group-containing organopolysiloxanes and reinforcing fillers;

l) from 0 to 10% by weight, preferably from 0 to 5% by weight, of said surfactants, emulsifiers and stabilizers;

m) from 0 to 90% by weight, preferably from 0 to 80% by weight, of said radiopaque substances;

n) from 0 to 20% by weight, preferably from 0 to 10% by weight, of said $H_2$-absorbers/adsorbers or substances which reduce or eliminate $H_2$ evolution.

31. Use of an addition-cross-linkable two-component silicone material as a sprayable material for checkbite recording, material for coating the occlusion fork, or recording plates in recording, tooth key materials, e.g., for intraoral support pivot recordings, transfer of brackets in orthodontics, remounting of ceramic, cast or plastic fillings for grinding the occlusion on the model, selfcuring rebasing silicone, modeling material for the preparation of inlays/onlays, having a Shore D hardness of greater than 35 and a modulus of elasticity of greater than 20 MPa as measured according to DIN 53457 or 53455 in the cured state, consisting of

a) organopolysiloxanes having two vinyl groups in the molecule and a viscosity of between 21 and 99 mPa·s (20 °C);

b) organohydrogenpolysiloxanes having two or more SiH groups in the molecule and an SiH content of from 1 to 15 mmol/g as cross-linking agents;

c) transition metals and transition metal compounds of the 8th subgroup as catalysts for accelerating the hydrosilylation reaction;

d) reinforcing fillers having a BET surface area of at least 50 m$^2$/g; and

e) non-reinforcing fillers.

**Revendications**

1. Matériau silicone à deux composants réticulant par addition contenant :

a) des organopolysiloxanes ayant des groupes vinyle dans la molécule,
b) comme agent de réticulation des organohydogénosiloxanes avec deux groupes SiH ou plus et un taux de SiH de 1 à 15 mmol/g,
c) comme catalyseur des métaux de transition et des composés de métaux de transition du sous-groupe 8,
d) des charges de renforcement avec une surface BET d'au moins 50 m$^2$/g,
e) des charges non renforçantes,

caractérisé en ce que la substance a) est un organopolysiloxane avec deux groupes vinyle dans la molécule ayant une viscosité comprise entre 21 et 99 mPa·s (20°C) et en ce que le matériau silicone présente une dureté Shore D supérieure à 35 et un module d'élasticité supérieur à 20 MPa (mesuré selon la norme DIN 53457 ou 53455) à l'état complètement vulcanisé.

2. Matériau silicone selon la revendication 1, caractérisé en ce que, les charges de renforcement selon le composant d) sont des charges actives, hautement dispersées avec une surface BET d'au moins 50 $m^2$/g comme le dioxyde de titane, l'oxyde d'aluminium, l'oxyde de zirconium, l'acide silicique précipité en solution ou obtenu de manière pyrogène, ce par quoi les matières citées se présentent sous forme hydrophile ou hydrophobe, ou contient des charges de renforcement en forme de fibres ou de paillettes, des charges minérales en forme de fibres, comme la wollastonite, et des charges synthétiques, en forme de fibres, comme des fibres de verre, des fibres de céramique ou des fibres de matières plastiques.

3. Matériau silicone selon la revendication 1, caractérisé en ce que les charges non renforçantes selon le composant e) sont des oxydes métalliques, des hydroxydes métalliques, des systèmes d'oxydes mixtes et des systèmes d'hydroxydes mixtes, de préférence le dioxyde de silicium, aussi sous la forme de quartz et de ses modifications cristallites, la silice vitreuse, l'oxyde d'aluminium, l'oxyde de calcium, l'hydroxyde d'aluminium, le carbonate de calcium, la diatomite, la terre à diatomées, le talc, le verre, des charges à base de matières synthétiques, le polyméthacrylate de méthyle, le polycarbonate, le polychlorure de vinyle, les poudres de résine de silicone, les poudres à base de composés organiques fluorés, ainsi que de billes creuses, de billes massives et de fibres organiques et inorganiques, ainsi que les particules de matière synthétique pleines ou creuses, aussi sous forme sphérique, sur la surface desquelles des charges inorganiques sont enrobées.

4. Matériau silicone selon l'une des revendications 1 à 3, caractérisé en ce que le matériau silicone contient f) des colorants.

5. Matériau silicone selon l'une des revendications 1 à 4, caractérisé en ce que le matériau silicone contient g) des absorbeurs d'humidité.

6. Matériau silicone selon l'une des revendications 1 à 5, caractérisé en ce que le matériau silicone contient h) des organopolysiloxanes avec plus de deux groupes vinyle dans la molécule.

7. Matériau silicone selon l'une des revendications 1 à 6, caractérisé en ce que le matériau silicone contient i) des inhibiteurs pour le réglage de la réactivité.

8. Matériau silicone selon l'une des revendications 1 à 7, caractérisé en ce que le matériau silicone contient j) des résines MQ solides ou liquides, contenant des groupes vinyle.

9. Matériau silicone selon l'une des revendications 1 à 8, caractérisé en ce que le matériau silicone contient k) des composés d'organopolysiloxanes et de charges de renforcement.

10. Matériau silicone selon l'une des revendications 1 à 9, caractérisé en ce que le matériau silicone contient i) des agents tensioactifs, émulsionnants et stabilisants.

11. Matériau silicone selon l'une des revendications 1 à 10, caractérisé en ce que le matériau silicone contient m) des substances radioopaques.

12. Matériau silicone selon l'une des revendications 1 à 11, caractérisé en ce que le matériau silicone contient n) des substances absorbantes ou absorbant $H_2$ et des substances, qui réduisent ou éliminent le dégagement de $H_2$.

13. Matériau silicone selon l'une des revendications 1 à 12, caractérisé en ce qu'il contient des organopolysiloxanes (a) avec deux groupes vinyle dans la molécule et une viscosité comprise entre 21 et 99 mPa·s (20°C) comprenant des substances de formule générale :

$$CH_2=CH-SiR_2O-(SiR_2O)_n-SiR_2-CH=CH_2,$$

dans laquelle

R  est un groupe alkyle (par exemple méthyle, éthyle, isopropyle), aryle (par exemple, phényle, naphtyle, tolyle, xylyle), aralkyle (benzyle, phényléthyle) et alkyle et aryle substitué par des halogènes (par exemple 3,3,3-trifluoropropyle, chlorophényle, difluorophényle), cyanoalkyle, cycloalkyle et cycloalcényle, de préférence R est le méthyle ;

n  est un nombre entier compris entre 21 et 69, « n » représentant une valeur théorique calculée, qui a été calculée à partir du taux de vinyle déterminé expérimentalement, en supposant qu'il y a deux groupes vinyle par molécule, et que R est le méthyle.

14. Matériau silicone selon l'une des revendications 1 à 13, caractérisé en ce que les composants organohydrogénopolysiloxane (b) sont comme agents de réticulation des polyalkyl-, polyaryl- et polyalkylaryl-ainsi que des polyhalogénoalkyl-, polyhalogénoaryl- et polyhalogénolalkylaryl-siloxanes, qui présentent dans la molécule au moins deux atomes d'hydrogène liés aux atomes de silicium, les agents de réticulation étant utilisés avec un taux de SiH compris entre 1 et 15 mmol/g.

15. Matériau silicone selon l'une des revendications 1 à 14, caractérisé en ce qu'on utilise comme catalyseurs (c) pour l'hydrosilylation des sels, des complexes et des formes colloïdales des métaux de transition du sous-groupe 8, de préférence les métaux platine, palladium et rhodium, en particulier les complexes du platine qui sont préparés par exemple à partir de l'acide hexachloroplatinique ou de sels de platine.

16. Matériau silicone selon l'une des revendications 1 à 15, caractérisé en ce que les charges (d) et (e) de renforcement et non renforçantes se présentent sous une forme traitée en surface (gecoated), dans laquelle on réalise le traitement de surface, par exemple avec des silanes et des acides gras, qui présentent des groupes fonctionnels comme le vinyle, l'allyle, -SiH.

17. Matériau silicone selon l'une des revendications 1 à 16, caractérisé en ce que les colorants (f) sont des colorants solubles ou des colorants de type pigments, des colorants alimentaires et des pâtes colorantes constitués de formulations de colorants et de polysiloxane ou d'huile minérale.

18. Matériau silicone selon l'une des revendications 1 à 17, caractérisé en ce qu'on utilise comme absorbeur d'humidité (g) des zéolites, le sulfate d'aluminium anhydre, du tamis moléculaire, la diatomite et le gel bleu.

19. Matériau silicone selon l'une des revendications 1 à 18, caractérisé en ce que les organopolysiloxanes avec plus de deux groupes vinyle dans la molécule (h) sont des organopolysiloxanes arrêtés par des groupes terminaux vinyle et ayant des chaînes latérales vinyle avec une viscosité de 21 à 350000 mPa·s (20°C).

20. Matériau silicone selon l'une des revendications 1 à 19, caractérisé en ce qu'on utilise comme inhibiteurs (i) pour ajuster la réactivité de la réaction d'hydrosilylation des organopolysiloxanes à chaîne courte de formule générale :

$$CH_2=CH\text{-}SiR_2O\text{-}(SiR_2O)_n\text{-}SiR_2\text{-}CH=CH_2,$$

dans laquelle

les R représentent des groupes hydrocarbonés identiques ou différents, éventuellement substitués, comme des groupes alkyle, alcényle, alcynyle et
n = 0 ou un nombre entier compris entre 1 et 6,
R étant en outre un radical siloxane à terminaison alcényle ou alcynyle.

21. Matériau silicone selon la revendication 20, caractérisé en ce que, pour la régulation de la vitesse de réticulation, on utilise des siloxanes cycliques vinyliques, le tétra-vinyltétraméthylcyclotétrasiloxane, des composés hydroxyle organiques contenant des doubles ou triples liaisons terminales.

22. Matériau silicone selon l'une des revendications 1 à 21, caractérisé en ce que les motifs Q contiennent le motif tétrafonctionnel $SiO_{4/2}$ et comme constituant M le motif monofonctionnel $R_3SiO_{1/2}$.

23. Matériau silicone selon l'une des revendications 1 à 22, caractérisé en ce que les résines MQ solides ou liquides contenant des groupes vinyle et éthoxy, contiennent comme motifs T le motif trifonctionnel $RSiO_{3/2}$ et comme motifs D le motif bifonctionnel $R_2SiO_{2/2}$, dans lequel R est un groupe vinyle, méthyle, éthyle, phényle.

**24.** Matériau silicone selon la revendication 23, caractérisé en ce que les résines MQ sont utilisées dissoutes dans des organopolysiloxanes avec deux groupes vinyle ou plus dans la molécule et une viscosité de 21 à 350000 mPa·s (20°C), le taux de groupes vinyle des résines MQ étant situé dans la gamme comprise entre 0,1 et 8 mmol/g et le taux de groupes éthoxy à moins de 4 mmol/g.

**25.** Matériau silicone selon la revendication 22, caractérisé en ce que le taux de SiOH des résines MQ est si faible qu'il ne se produit pas de bouillonnement par dégagement d'hydrogène, le taux de composants volatils des résines MQ étant si faible que la stabilité dimensionnelle n'est pas affectée.

**26.** Matériau silicone selon l'une des revendications 1 à 25, caractérisé en ce que les composés (k) sont constitués d'organopolysiloxanes avec deux groupes vinyle ou plus dans la molécule et une viscosité de 21 à 350000 mPa·s et des additifs de renforcement, ceux-ci étant rendus hydrophobes en utilisant des adjuvants modifiants, par exemple l'hexaméthyldisilazane in situ.

**27.** Matériau silicone selon l'une des revendications 1 à 26, caractérisé en ce qu'il s'agit pour les composants (1) utilisés comme agents tensioactifs, émulsionnants et stabilisants d'agents tensioactifs anioniques, en particulier d'alkylsulfates, d'alkylbenzènesulfonates et -phosphates, d'agents tensioactifs cationiques, d'halogénures de tétraalkylammonium, d'agents tensioactifs non ioniques, en particulier les alkyl- et alkylphényl-polyalkyloxyalkylène et leurs éthers d'alkyle et esters d'alkyle, les alkylolamides d'acides gras, les esters de saccharose avec un acide gras, les trialkylaminooxydes, les agents tensioactifs silicone ou les agents tensioactifs fluorés ainsi que les agents tensioactifs amphotères, les produits de condensation sulfatés ou éthoxylés d'alkylènephénols et de formaldéhyde, les polymérisats séquencés oxyéthylèneoxypropylène et les polysiloxanes modifiés, les agents tensioactifs contenant encore des groupes fonctionnels comme -OH, -CH=CH$_2$, -OCO-(CH$_3$)C=CH$_2$ et SiH.

**28.** Matériau silicone selon l'une des revendications 1 à 27, caractérisé en ce qu'on utilise comme substances radioopaques des verres contenant du baryum, du strontium, du lanthane ou du zinc, le sulfate de baryum, le dioxyde de zirconium, l'oxyde de lanthane ou des compositions de substances céramique, les oxydes de lanthane, d'hafnium et de métaux alcalino-terreux, les fluorures complexes de métaux lourds de formule générale M$^{II}$M$^{IV}$F$_6$ ou YF$_3$, dans lesquelles M$^{II}$ représente un ion calcium, strontium ou baryum et M$^{IV}$ un ion titane, zirconium, ou hafnium, ainsi que des groupes d'atomes ou des atomes liés au polymère silicone, qui possèdent des caractéristiques radioopaques, comme de l'iode liée à du silicium.

**29.** Matériau silicone selon l'une des revendications 1 à 28, caractérisé en ce qu'il s'agit pour les absorbants/absorbants de H$_2$ cités en (n) de palladium ou de platine finement divisé ou de leurs alliages , qui sont contenus dans des aluminosilicates, ainsi que de substances qui éliminent ou réduisent le dégagement de H$_2$, comme le 3-méthyl-1-butyn-3-ol et CH$_3$Si[O-C(CH$_3$)$_2$-C$\equiv$CH]$_3$.

**30.** Matériau silicone selon l'une des revendications 1 à 29, caractérisé en ce le matériau silicone contient les quantités suivantes de matériau silicone des composants individuels (a) à (n) en % en poids, par rapport à l'ensemble du matériau silicone :

a) de 1 à 90% en poids, de préférence de 10 à 60% en poids d'organopolysiloxanes avec deux groupes vinyle dans la molécule et une viscosité comprise entre 21 et 99 mPa·s (20°C),
b) de 1 à 40% en poids, de préférence, de 1 à 20% en poids d'organohydrogénopolysiloxanes,
c) de 0,0001 à 0,1% en poids, de préférence de 0,0005 à 0,1% en poids de catalyseurs pour accélérer la réaction d'hydrosilylation, par rapport au métal pur,
d) jusqu'à 80% en poids, de préférence jusqu'à 50% en poids de charges de renforcement,
e) jusqu'à 90% en poids, de préférence de 20 à 80% en poids de charges non renforçantes,
f) de 0 à 5% en poids, de préférence de 0 à 2% en poids de colorants,
g) de 0 à 30% en poids, de préférence de 0 à 5% en poids d'absorbeur d'humidité,
h) de 0 à 70% en poids, de préférence de 0 à 20% en poids d'organopolysiloxanes avec plus de deux groupes vinyle par molécule,
i) de 0 à 1,0% en poids, de préférence de 0 à 0,1% en poids d'inhibiteurs,
j) de 0 à 90% en poids, de préférence de 0 à 50% en poids de résine MQ contenant des groupes vinyle,
k) de 0 à 80% en poids, de préférence de 0 à 50% en poids du composé d'organopolysiloxanes contenant des groupes vinyle avec des charges de renforcement,
l) de 0 à 10% en poids, de préférence de 0 à 5% en poids d'agents tensioactifs, émulsionnants et stabilisants,
m) de 0 à 90% en poids, de préférence de 0 à 80% en poids de substances radioopaques,

n) de 0 à 20% en poids, de préférence de 0 à 10% en poids d'absorbant/absorbant de $H_2$ ou de substances réduisant ou éliminant le dégagement de $H_2$.

31. Utilisation d'un matériau silicone à 2 composants réticulant par addition comme matériau pulvérisable pour la prise d'empreinte de la mâchoire ou le revêtement de la cuillère à empreinte ou des plaques d'enregistrement lors de la prise d'empreinte, matériau clé, par exemple pour la prise d'empreinte intraorale de broche en appui, le transfert de crochets dans l'orthopédie maxillaire, le remontage d'obturations de céramiques, de coulages ou de matières synthétiques pour la fermeture de l'occlusion sur le modèle, silicone de doublage durcissante, matériau de modèle pour la préparation de Inlays/Onlays, avec une dureté Shore D supérieure à 35 et un module d'élasticité supérieur à 20 MPa mesuré selon la norme DIN 53457 ou DIN 53455 à l'état complètement vulcanisé, constitué

a) d'organopolysiloxanes avec deux groupes vinyle dans la molécule et avec une viscosité comprise entre 21 et 99 mPa·s (20°C),
b) d'organohydrogénopolysiloxanes comme agent réticulant avec deux groupes SiH ou plus dans la molécule et avec une teneur en SiH de 1 à 15 mmol/g,
c) de métaux de transition et de composés de métaux de transition du sous-groupe 8 comme catalyseurs pour l'accélération de la réaction d'hydrosilylation,
d) de charges de renforcement avec une surface BET d'au moins 50 $m^2$/g
et
e) de charges non renforçantes.

EP 0 894 117 B1

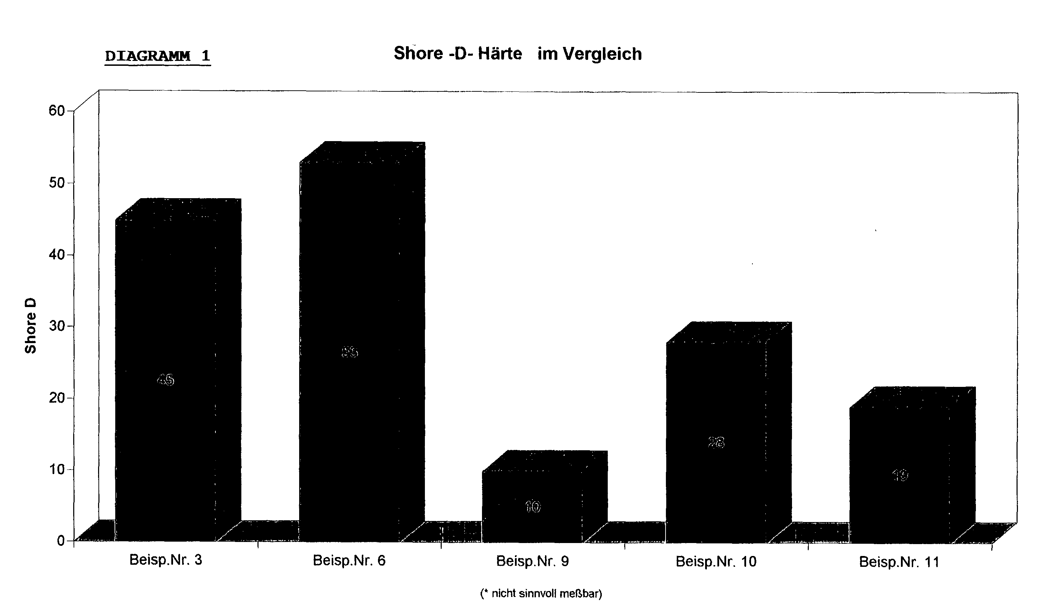

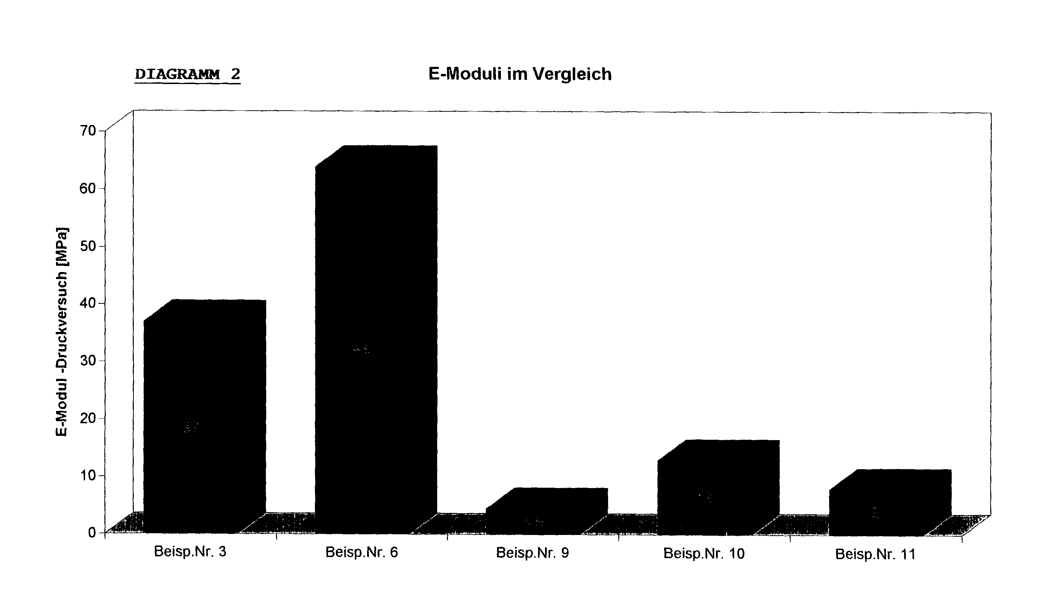

**DIAGRAMM 1**       **Shore -D- Härte   im Vergleich**

Shore D

60
50
40
30
20
10
0

Beisp.Nr. 3    Beisp.Nr. 6    Beisp.Nr. 9    Beisp.Nr. 10    Beisp.Nr. 11

(* nicht sinnvoll meßbar)

DIAGRAMM 2

E-Moduli im Vergleich